# EUROPEAN PATENT APPLICATION

(11) **EP 4 742 256 A1**
(43) Date of publication of application: **13.05.2026**
(21) Application number: 25212894.7
(22) Date of filing: 03.11.2025
(51) Int. Cl.: G16H 10/60, G16H 40/40

(54) **AUTOMATED INTELLIGENT ACTIONABLE EDIT SYSTEM USING MACHINE LEARNING**

(30) Priority: 06.11.2024 US 202463717013 P
(71) Applicant: Solventum Intellectual Properties Company, Maplewood, MN 55144 (US)
(72) Inventor: Naef, Jeramie Paul, Maplewood, MN, 55144 (US)
(74) Representative: Vossius & Partner Patentanwälte Rechtsanwälte mbB

(57) **Abstract**

Techniques for recommending actions for resolution of errors associated with medical coding records are described. In an example, a plurality of edits corresponding to a medical coding record is identified, where the plurality of edits is indicative of resolutions to at least one error associated with the medical coding record. The plurality of edits is then analysed using a code editing model to rank the plurality of edits, where the code editing model is trained based on the statistical information indicative of relevance of the plurality of edits for resolution of errors associated with the medical coding record. The ranked plurality of edits is then provided along with a set of recommendation actions for resolution of the error associated with the medical coding record.

## Description

### BACKGROUND

Healthcare practitioners provide numerous services depending on the patient's requirements. To keep track of the services provided to a patient, a medical chart is prepared for the patient, where the medical chart tracks information of every medical diagnosis and treatment performed on the patient. Edits to the medical chart can be made by specialists to address issues in the medical chart.

### BRIEF DESCRIPTION OF DRAWINGS

The following detailed description references the drawings, wherein:
Figure 1 illustrates a computing system for recommending actions for resolution of errors associated with medical coding records, in accordance with an example of the present subject matter,
Figure 2 illustrates the computing system for recommending actions for resolution of errors associated with medical coding records, in accordance with another example of the present subject matter,
Figure 3 illustrates a method for recommending actions for resolution of errors associated with medical coding records, in accordance with an example of the present subject matter, and
Figure 4 illustrates a non-transitory computer-readable medium for recommending actions for resolution of errors associated with medical coding records, in accordance with an example of the present subject matter.

Throughout the drawings, identical reference numbers designate similar, but not necessarily identical, elements. The drawings provide examples and/or implementations consistent with the description; however, the description is not limited to the examples and/or implementations provided in the drawings.

### DETAILED DESCRIPTION

Medical coding records may need to be modified for various reasons. In operation, the medical coding records are usually identified manually by referring to a list that includes various medical coding records along with their respective descriptions. However, manual identification and inclusion of the medical coding records is prone to errors. If ignored, such errors can prove costly for the providers and my ultimately impact the quality of medical care. For instance, the inclusion of an incorrect medical coding record in an invoice may lead to rejection of insurance claims for reimbursement by an insurance company. As another example, the misidentification of a medical code may prevent systems specifically configured to address preventable complications from presenting relevant information to a medical professional, leading to increased risk of complications or other adverse medical outcomes.

To alleviate the above-mentioned problems, a practice of editing the medical coding records is adopted, where potential errors associated with a medical coding record are identified and various edits that could be made to the medical coding records are presented to the users. However, in known claim editing processes, a user is usually presented with numerous edits, such that, the user may have to sift through numerous edits for identification of a relevant edit. Sifting through such numerous edits causes edit fatigue as it becomes difficult to know which edits are important to the user. Accordingly, the user tends to get confused as to which edit should be addressed first. Further, the user may also at times choose to ignore the presented edits as finding the solution to the edit can be tedious, challenging and time consuming. All of these factors contribute to errors in conventional editing techniques.

According to examples of the present subject matter, techniques for recommending actions for resolution of errors associated with medical coding records are described.

In operation, a plurality of edits corresponding to a medical coding record are identified, where the plurality of edits is indicative of resolutions to at least one error associated with the medical coding record. The plurality of edits is then analysed using a code editing model to evaluate the plurality of edits, where the code editing model is trained based on the statistical information indicative of relevance of the plurality of edits for resolution of errors associated with the medical coding record. In an example, the evaluation of the plurality of edits involves computing a confidence score for each of the plurality of edits and ranking each of the plurality of edits based on the confidence score. The ranked plurality of edits is then provided along with a set of recommendation actions for resolution of the error associated with the medical coding record.

Ranking the plurality of edits based on the statistical information and providing the ranked plurality of edits along with the set of recommended actions facilitates a user in initiating an appropriate action for resolution of errors associated with medical coding records, thereby improving the efficiency involved in resolution of the errors associated with the medical coding records.

The above aspects are further described in conjunction with the figures, and in the associated description below. It should be noted that the description and figures merely illustrate principles of the present subject matter. Therefore, various arrangements that encompass the principles of the present subject matter, although not explicitly described or shown herein, may be devised from the description, and are included within its scope.

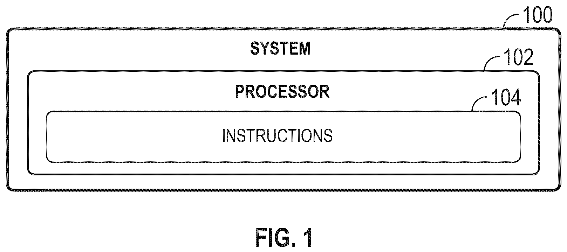

Figure 1 illustrates a computing system 100 for recommending actions for resolution of errors associated with medical coding records, in accordance with an example of the present subject matter.

The computing system 100 may include include a processor 102. In an example, the processor 102 may fetch and execute the computer-readable instructions 104 stored in a memory (not depicted in Figure 1), for recommending actions for resolution of errors associated with medical coding records, amongst other functions.

In operation, the processor 102 may cause the computing system 100 to identify a plurality of edits corresponding to a medical coding record. The plurality of edits may be indicative of resolutions to at least one error associated with the medical coding record. In an example, the medical coding record may be indicative of at least one service received by a patient at a medical facility.

The processor 102 may then cause the computing system 100 to analyse the plurality of edits using a code editing model to rank the plurality of edits. In an example, the code editing model may be a machine learning model trained based on the statistical information indicative of relevance of the plurality of edits for resolution of errors associated with the medical coding record.

The processor 102 may subsequently cause the computing system 100 to provide the ranked plurality of edits. In an example, the processor 102 may cause the computing system 100 to provide the ranked plurality of edits along with a set of recommendation actions for resolution of the error associated with the medical coding record.

Ranking the plurality of edits based on the statistical information and providing the ranked plurality of edits along with the set of recommendation actions facilitates a user in initiating an appropriate action for resolution of errors associated with medical coding records, thereby improving the efficiency involved in resolution of the errors associated with the medical coding records.

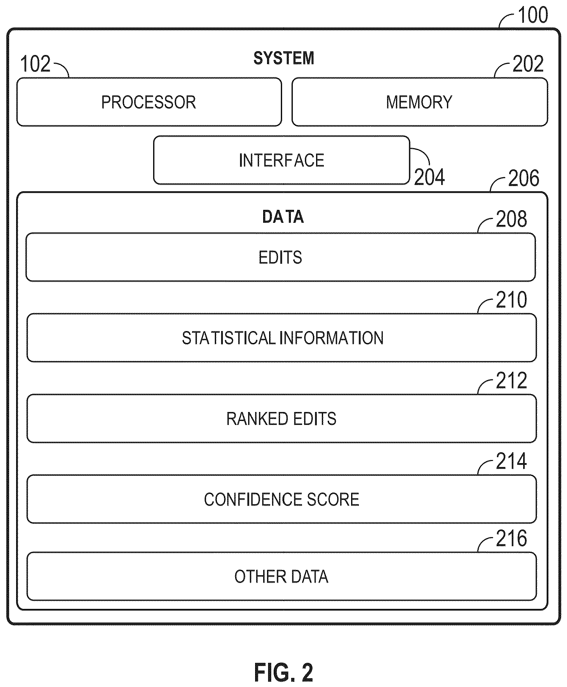

Figure 2 illustrates the computing system 100 for recommending actions for resolution of errors associated with medical coding records, in accordance with another example of the present subject matter.

The computing system 100 may include the processor 102, memory 202 coupled to the processor 102, and the interface 204 coupled to the memory 202. The functions of various elements shown in the figures, including any functional blocks labelled as "processor(s)", may be provided using dedicated hardware as well as hardware capable of executing instructions. When provided by a processor, the functions may be provided by a single dedicated processor, by a single shared processor, or by a plurality of individual processors, some of which may be shared. Moreover, explicit use of the term "processor" would not be construed to refer exclusively to hardware capable of executing instructions, and may implicitly include, without limitation, digital signal processor (DSP) hardware, network processor, application specific integrated circuit (ASIC), field programmable gate array (FPGA). Other hardware, standard and/or custom, may also be coupled to the processor 102.

The memory 202 may be a computer-readable medium, examples of which include volatile memory (e.g., RAM), and/or non-volatile memory (e.g., Erasable Programmable read-only memory, i.e., EPROM, flash memory, etc.). The memory 202 may be an external memory, or internal memory, such as a flash drive, a compact disk drive, an external hard disk drive, or the like. The memory 202 may further include data which either may be utilized or generated during the operation of the computing system 100.

The interface 204 may allow the connection or coupling of the CU-CP 112 with one or more other devices, through a wired (e.g., Local Area Network, i.e., LAN) connection or through a wireless connection (e.g., BLUETOOTH Wi-Fi). The interface 204 may also enable intercommunication between different logical as well as hardware components of the computing system 100.

The computing system 100 may further include data 206 that may be utilized or generated by the processor 102 while performing a variety of functions. In an example, the data 206 includes edits 208, statistical information 210, ranked edits 212, confidence score 214, and other data 216. The other data 216, amongst other things, may serve as a repository for storing data that is processed, or received, or generated because of the execution of the instructions by the processor 102.

In operation, the processor 102 may cause the computing system 100 to identify a plurality of edits corresponding to a medical coding record. The plurality of edits may be indicative of resolutions to at least one error associated with the medical coding record. In an example, the medical coding record may be indicative of at least one service availed by a patient at a medical facility.

The processor 102 may then cause the computing system 100 to analyse the plurality of edits using a code editing model to rank the plurality of edits. In an example, the processor 102 may train the code editing model based on the statistical information indicative of relevance of the plurality of edits for resolution of errors associated with the medical coding record. In the example, the statistical information may include contextual information associated with the plurality of edits. For instance, the contextual information may include a category of the plurality of edits, a severity level of the plurality of edits, an experience level of a coder who created the plurality of edits, a state of resolution indicating whether the plurality of edits led to resolution of the at least one error associated with medical coding record, a count of utilization of the plurality of edits for resolution of the at least one error associated with medical coding record, or a combination thereof.

Each of the plurality of edits may have at least one of an edit identifiers and edit text. The edit identifier may uniquely identify an edit amongst the plurality of edits. Further, the edit text may be a label for further identifying the edit amongst the plurality of edits. In an example, the contextual information corresponding to each of the plurality of edits may be stored along with the edit identifier and corresponding edit text. In the example, the edit identifier may be based at least in part on one or more parameters included in text of the edit. The one or more parameters may include at least a numerical value and a classification title, where the classification title may specify an edit type and the numerical value may be unique for each edit type.

There may be a situation when the statistical information may not include contextual information corresponding to at least one edit from the plurality of edits. In an example, the processor 102 may determine whether the statistical information includes contextual information corresponding to the at least one edit from the plurality of edits based on presence of an edit identifier associated with the at least one edit in the statistical information. In an example, the processor 102 may determine that the statistical information does not include contextual information corresponding to the at least one edit. In such a situation, the processor 102 may generate the contextual information corresponding to the at least one edit.

In an example, to generate the contextual information corresponding to the at least one edit, the processor 102 may analyse text of the at least one edit to identify a correlation between the at least one edit and the error associated with the medical coding record. In an example, the processor 102 may analyse the text of the at least one edit based on Natural Language Processing (NLP). To analyse the text of the at least one edit, the processor 102 may parse the text of the at least edit into one or more segments that correspond to at least one other edit indicative of a resolution to the at least one error associated with the medical coding record. In an example, the at least one other edit may be mutually exclusive with respect to the plurality of edits.

Upon identification of the correlation, the processor 102 may identify instances when the at least one other edit was utilized for resolution of the error in the past. Based on the identification of such instances, the processor 102 may generate contextual information corresponding to the at least one edit. Particularly, the processor 102 may identify a severity level of the at least one edit with respect to the error, an experience level of a coder who created the at least one edit for resolution of the error, a state of resolution indicating whether the at least one edit led to resolution of the error, a count of utilization of the at least one edit for resolution of the error, or a combination thereof.

In another example, the processor 102 may determine that the statistical information includes contextual information corresponding to the at least one edit. In such a situation, the processor 102 may compare the edit identifier associated with the at least one edit with the edit identifiers included in the statistical information.

In an example, upon comparison of the edit identifier associated with the at least one edit with the edit identifiers included in the statistical information, the processor 102 may determine that the edit identifier associated with the at least one edit matches an edit identifier associated with a first edit from the plurality of edits. In such a situation, the processor 102 may compare text of the at least one edit with text of the first edit to confirm that the contextual information corresponding to the at least one edit is indeed present in the statistical information.

In another example, upon comparison of the edit identifier associated with the at least one edit with the edit identifiers included in the statistical information, the processor 102 may determine that the edit identifier associated with the at least one edit is not one of the edit identifiers included in the statistical information. In such a situation, the processor 102 may generate the contextual information corresponding to the at least one edit. The way the processor 102 generates the contextual information corresponding to the at least one edit is described above and is not being redescribed for the sake of brevity.

The processor 102 may subsequently cause the computing system 100 to compute a confidence score for each of the plurality of edits. The processor 102 may cause the computing system 100 to compute the confidence score for each of the plurality of edits based on the contextual information. The processor 102 may then rank each of the plurality of edits based on the confidence score. Thereafter, the processor 102 may cause the computing system 100 to provide the ranked plurality of edits. In an example, the processor 102 may cause the computing system 100 to provide the ranked plurality of edits along with a set of recommendation actions for resolution of the error associated with the medical coding record.

In an example, the processor 102 may then cause the computing system 100 to receive user selection of at least one recommended action from the set of recommended actions. In the example, the processor 102 may update the statistical information to include the user selection of the at least one recommended action for resolution of the error associated with the medical coding record. Further, in the example, the processor 102 may train the code editing model based on the updated statistical information.

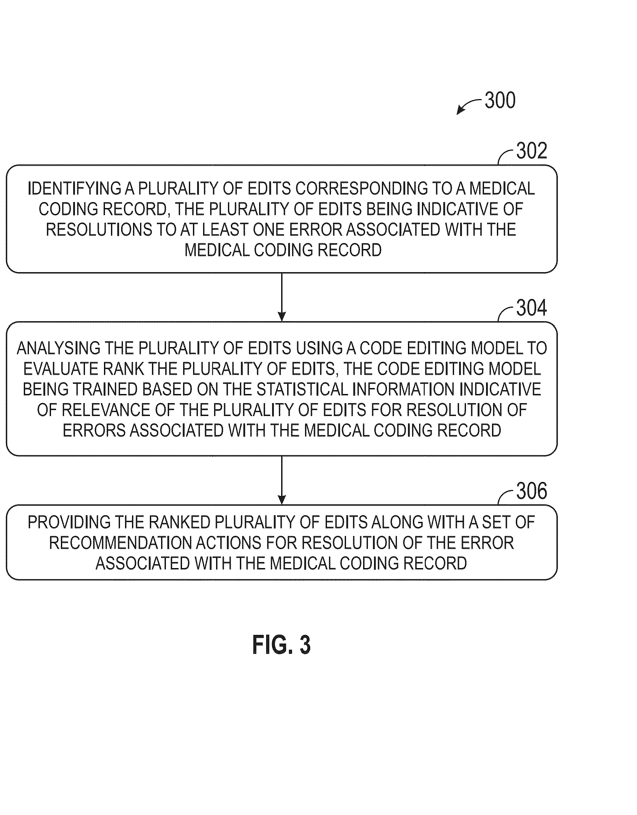

Figure 3 illustrates a method 300 for recommending actions for resolution of errors associated with medical coding records, in accordance with examples of the present subject matter. The order in which the method is described is not intended to be construed as a limitation, and any number of the described method blocks may be combined in any order to implement the methods, or an alternative method. Further, the method 300 may be implemented by processing resource or computing device(s) through any suitable hardware, non-transitory machine-readable instructions, or combination thereof.

It may also be understood that method 300 may be performed by programmed computing device, such as the computing system 100, as depicted in Figure 2. Furthermore, the method 300 may be executed based on instructions stored in a non-transitory computer readable medium, as will be readily understood. The non-transitory computer readable medium may include, for example, digital memories, magnetic storage media, such as one or more magnetic disks and magnetic tapes, hard drives, or optically readable digital data storage media. The method 300 is described below with reference to the computing system 100, as described above; other suitable computing systems for the execution of these methods may also be utilized. Additionally, implementation of the method is not limited to such examples.

At block 302, a plurality of edits corresponding to a medical coding record may be identified, where the plurality of edits may be indicative of resolutions to at least one error associated with the medical coding record.

At block 304, the plurality of edits may be analysed using a code editing model to rank the plurality of edits, where the code editing model is trained based on statistical information indicative of relevance of the plurality of edits for resolution of errors associated with the medical coding record. The statistical information may include contextual information associated with the plurality of edits, where the contextual information includes at least one of: a category of the plurality of edits, a severity level of the plurality of edits, an experience level of a coder who created the plurality of edits, a state of resolution indicating whether the plurality of edits led to resolution of the at least one error associated with medical coding record, and a count of utilization of the plurality of edits for resolution of the at least one error associated with medical coding record. Further, each of the plurality of edits may have an edit identifier and an edit text corresponding to the edit identifier. The edit identifier may uniquely identify an edit amongst the plurality of edits. Further, the edit text may be a label for further identifying the edit amongst the plurality of edits. In an example, the contextual information corresponding to the plurality of edits may be stored along with edit identifiers and corresponding edit text for the plurality of edits. In the example, the edit identifier may be based at least in part on one or more parameters included in text of the edit. The one or more parameters may include at least a numerical value and a classification title, where the classification title may specify an edit type and the numerical value may be unique for each edit type.

There may be a situation when the statistical information may not include contextual information corresponding to at least one edit from the plurality of edits. In an example, it may be determined whether the statistical information includes contextual information corresponding to the at least one edit from the plurality of edits based on presence of an edit identifier associated with the at least one edit in the statistical information. If it is determined that the statistical information does not include contextual information corresponding to the at least one edit, the contextual information corresponding to the at least one edit may be generated.

In an example, to generate the contextual information corresponding to the at least one edit, the text of the at least one edit may be analysed to identify a correlation between the at least one edit and the error associated with the medical coding record. In an example, the text of the at least one edit may be analysed based on Natural Language Processing (NLP). The text of the at least one edit may be analysed by parsing the text of the at least edit into one or more segments that correspond to at least one other edit indicative of a resolution to the at least one error associated with the medical coding record. In an example, the at least one other edit may be mutually exclusive with respect to the plurality of edits.

Upon identification of the correlation, instances may be identified when the at least one other edit was utilized for resolution of the error in the past. Based on the identification of such instances, contextual information of the at least one edit may be generated, where the contextual information may include severity level of the at least one edit with respect to the error, an experience level of a coder who created the at least one edit for resolution of the error, a state of resolution indicating whether at least one edit led to resolution of the error, a count of utilization of the at least one edit for resolution of the error, or a combination thereof.

In another example, it may be determined that the statistical information includes contextual information corresponding to the at least one edit. In such a situation, the edit identifier associated with the at least one edit may be compared with the edit identifiers included in the statistical information.

In an example, upon comparison of the edit identifier associated with the at least one edit with the edit identifiers included in the statistical information, it may be determined that the edit identifier associated with the at least one edit matches an edit identifier associated with a first edit from the plurality of edits. In such a situation, the text of the at least one edit may be compared with text of the first edit to confirm that the contextual information corresponding to the at least one edit is indeed present in the statistical information.

In another example, upon comparison of the edit identifier associated with the at least one edit with the edit identifiers included in the statistical information, it may be determined that the edit identifier associated with the at least one edit is not one of the edit identifiers included in the statistical information. In such a situation, the contextual information corresponding to the at least one edit may be generated. The way the contextual information corresponding to the at least one edit is generated is described above and is not being redescribed for the sake of brevity.

In an example, once the contextual information corresponding to each of the plurality of edits is available, a confidence score for each of the plurality of edits may be generated based on the contextual information and the plurality of edits may be ranked based on the confidence score.

At block 306, the ranked plurality of edits may be provided. In an example, the ranked plurality of edits may be provided along with a set of recommended actions for resolution of the error associated with the medical coding record. The recommended actions could include automatic resolution of edits without user interaction, walking through possible recommended choices by asking questions, not showing low priority plurality of edits, giving a list of possible solutions that others have used, and other ways of providing valuable information.

In an example, a user selection of at least one recommended action from the set of recommended actions may be received. In the example, the statistical information may be updated to include the user selection of the at least one recommendation action for resolution of the error associated with the medical coding record. Further, in the example, the code editing model may be trained based on the updated statistical information.

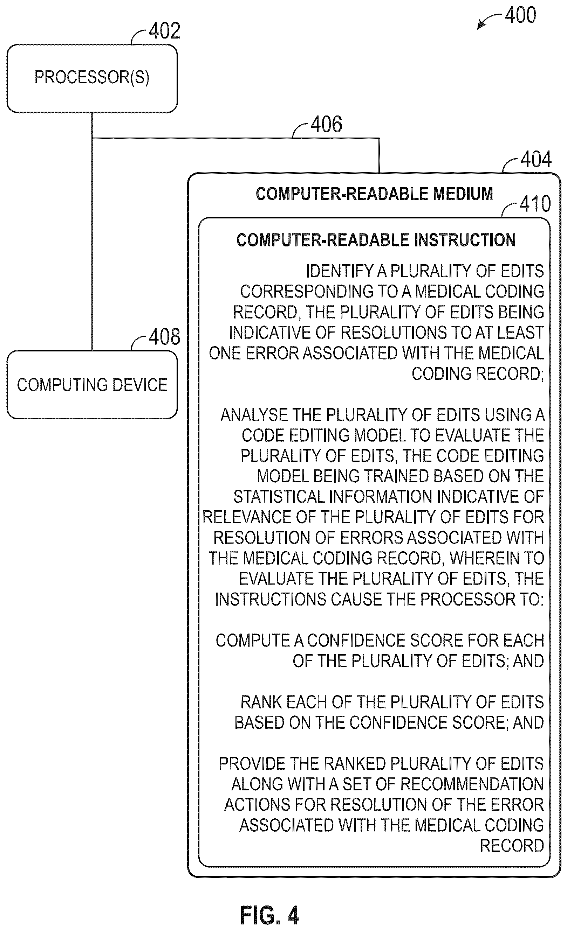

Figure 4 illustrates a non-transitory computer-readable medium for recommending actions for resolution of errors associated with medical coding records, in accordance with an example of the present subject matter.

In an example, the computing environment 400 includes processor 402 communicatively coupled to a non-transitory computer readable medium 404 through communication link 406. In an example implementation, the computing environment 400 may be for example, the computing system 100. In an example, the processor 402 may have one or more processing resources for fetching and executing computer-readable instructions from the non-transitory computer readable medium 404. The processor 402 and the non-transitory computer readable medium 404 may be implemented, for example, in the computing system 100.

The non-transitory computer readable medium 404 may be, for example, an internal memory device or an external memory. In an example implementation, the communication link 406 may be a network communication link, or other communication links, such as a PCI (Peripheral component interconnect) Express, USB-C (Universal Serial Bus Type-C) interfaces, I2C (Inter-Integrated Circuit) interfaces, etc. In an example implementation, the non-transitory computer readable medium 404 includes a set of computer readable instructions 410 which may be accessed by the processor 402 through the communication link 406 and subsequently executed for recommending actions for resolution of errors associated with medical coding records. The processor(s) 402 and the non-transitory computer readable medium 404 may also be communicatively coupled to a computing device 408 over the network.

Referring to Fig. 4, in an example, the non-transitory computer readable medium 404 includes computer readable instructions 410 that cause the processor 402 to identify a plurality of edits corresponding to a medical coding record, where the plurality of edits is indicative of resolutions to at least one error associated with the medical coding record.

The computer readable instructions 410 further cause the processor 402 to analyse the plurality of edits using a code editing model to evaluate the plurality of edits, where the code editing model is trained based on the statistical information indicative of relevance of the plurality of edits for resolution of errors associated with the medical coding record. In an example, to evaluate the plurality of edits, the computer readable instructions 410 cause the processor 402 to compute a confidence score for each of the plurality of edits, and rank each of the plurality of edits based on the confidence score.,

The computer readable instructions 410 subsequently cause the processor 402 to provide the ranked plurality of edits along with a set of recommendation actions for resolution of the error associated with the medical coding record.

Ranking the plurality of edits based on the statistical information and providing the ranked plurality of edits along with the set of recommended actions facilitates a user in initiating an appropriate action for resolution of errors associated with medical coding records, thereby improving the efficiency involved in resolution of the errors associated with the medical coding records.

Implementations of the present disclosure provide a number of advantages over conventional techniques. In particular, systems and techniques of the present disclosure improve the accuracy of the underlying computer system and do so in a way that cannot be replicated in the human mind with or without the aid of pen and paper. For instance, systems of the present disclosure ingest edits from a large number of sources that could be the equivalent of years or tens of years of human-based experiences. Utilizing this large corpus of information, the system of the present disclosure can evaluate the accuracy of edits in real-time or near-real-time to identify missing information concerning potential complications or other medically relevant information and it would be near impossible for a human (or multiple humans) to process the volume of information present and provide an analysis in a time-constrained manner while the patient may still be in the hospital. As a result, systems and techniques of the present disclosure may also improve the provision of medical care to patients by automatically identifying potential complications.

Another advantage of the present disclosure is that the machine learning, natural language processing, and automated intelligence generates a computer-based assistant that can improve the accuracy of the underlying computer system because the assistant gets smarter as it is used. This allows the system to automatically resolve edits, ask appropriate questions to resolve other important edits, present only edits that matter and place other edits in the background, and provide other features designed to detect and correct edit errors or other surface information the may directly impact patient care. Relatedly, the present disclosure also has advantages over traditional rule-based approaches because, over time, it will adapt to the needs to the specific hospital or facility. In short, systems of the present disclosure are more accurate than existing systems and without requiring the technical challenge of configuring the system to each specific use case. Instead, the system can adapt the use case without additional human intervention.

Although examples of the present subject matter have been described in language specific to methods and/or structural features, it is to be understood that the present subject matter is not limited to the specific methods or features described. Rather, the methods and specific features are disclosed and explained as examples of the present subject matter.

## Claims

1. A system comprising:
at least one processor;
a computer-readable medium comprising instructions that, when executed by the at least one processor, causes the system to:
identify a plurality of edits corresponding to a medical coding record, the plurality of edits being indicative of resolutions to at least one error associated with the medical coding record;
analyse the plurality of edits using a code editing model to rank the plurality of edits, the code editing model being trained based on the statistical information indicative of relevance of the plurality of edits for resolution of errors associated with the medical coding record; and
provide the ranked plurality of edits along with a set of recommended actions for resolution of the error associated with the medical coding record.

2. The system of claim 1, wherein to rank the plurality of edits, the at least one processor causes the system to:
compute a confidence score for each of the plurality of edits; and
rank each of the plurality of edits based on the confidence score.

3. The system of any of claims 1-2, wherein the statistical information comprises contextual information associated with the plurality of edits, the contextual information including at least one of: a category of the plurality of edits, a severity level of the plurality of edits, an experience level of a coder who created the plurality of edits, a state of resolution indicating whether the plurality of edits led to resolution of the at least one error associated with medical coding record, and a count of utilization of the plurality of edits for resolution of the at least one error associated with medical coding record.

4. The system of any of claims 1-3, wherein the at least one processor further causes the system to:
determine whether the statistical information includes the contextual information corresponding to at least one edit from the plurality of edits; and
generate the contextual information for the at least one edit based on determining that the statistical information does not include the contextual information corresponding to the at least one edit.

5. The system of any of claims 1-4, wherein the at least one processor further causes the system to analyse text of the at least one edit to identify a correlation between the at least one edit and the error associated with the medical coding record.

6. The system of any of claims 1-5, wherein to analyse the text of the at least edit, the processor causes the system to:
parse the text into one or more segments that correspond to at least one other edit indicative of a resolution to the at least one error associated with the medical coding record; and
generate contextual information corresponding to at least one edit based on past instances of utilization of the at least one other edit for the resolution to the at least one error.

7. The system of any of claims 1-6, wherein to generate the contextual information for the at least one edit, the processor causes the system to:
generate an edit identifier for each edit, wherein the edit identifier uniquely identifies the edit, and the edit identifier is based at least in part on one or more parameters included in text of the edit,
wherein the one or more parameters comprise at least a numerical parameter and a classification title, and wherein the classification title specifies an edit type, and the numerical value is unique for edits of each edit type.

8. The system of any of claims 1-7, wherein the at least one processor further causes the system to determine whether the statistical information includes the contextual information corresponding to at least one edit based on presence of an edit identifier associated with the at least one edit.

9. The of any of claims 1-8, wherein the at least one processor further causes the system to:
determine whether the edit identifier associated with the at least one edit matches an edit identifier associated with a first edit in the statistical information; and
compare text of the at least one edit with text of the first edit on determining that the edit identifier associated with the at least one edit matches the edit identifier associated with the first edit.

10. The system of claim 1, wherein the at least one processor further causes the system to:
receive a user selection of at least one recommended action from the set of recommended actions;
update the statistical information to include the user selection of the at least one recommended action for resolution of the error associated with the medical coding record; and
train the code editing model based on the updated statistical information.

11. A computer implemented method comprising:
identifying, by a medical code editing system, a plurality of edits corresponding to a medical coding record, the plurality of edits being indicative of resolutions to at least one error associated with the medical coding record;
analysing, by the medical code editing system, the plurality of edits using a code editing model to rank the plurality of edits, the code editing model being trained based on the statistical information indicative of relevance of the plurality of edits for resolution of errors associated with the medical coding record; 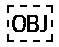
providing, by the medical code editing system, the ranked plurality of edits along with a set of recommended actions for resolution of the error associated with the medical coding record.

12. The method of claim 11, wherein ranking the plurality of edits comprises:
computing a confidence score for each of the plurality of edits; and
ranking each of the plurality of edits based on the confidence score.

13. The method of any of claims 11-13, wherein the statistical information comprises contextual information associated with the plurality of edits, the contextual information including at least one of: a category of the plurality of edits, a severity level of the plurality of edits, an experience level of a coder who created the plurality of edits, a state of resolution indicating whether the plurality of edits led to resolution of the at least one error associated with medical coding record, and a count of utilization of the plurality of edits for resolution of the at least one error associated with medical coding record.

14. The method of any of claims 11-13, further comprising:
determining whether the statistical information includes the contextual information corresponding to at least one edit from the plurality of edits; and
generating the contextual information for the at least one edit based on determining that the statistical information does not include the contextual information corresponding to the at least one edit.

15. The method of any of claims 11-14, wherein generating the contextual information comprises analysing text of the at least one edit to identify a correlation between the at least one edit and the error associated with the medical coding record, wherein the text of the at least one edit is analysed based on natural language processing (NLP).
